(19) Europäisches Patentamt
European Patent Office
Office européen des brevets

(11) Veröffentlichungsnummer: **0 175 290**
A2

(12) # EUROPÄISCHE PATENTANMELDUNG

(21) Anmeldenummer: **85111557.6**

(22) Anmeldetag: **12.09.85**

(51) Int. Cl.⁴: **C 07 K 9/00**, A 61 K 37/02

(30) Priorität: **17.09.84 DE 3434039**

(43) Veröffentlichungstag der Anmeldung: **26.03.86 Patentblatt 86/13**

(84) Benannte Vertragsstaaten: **AT BE CH DE FR GB IT LI LU NL SE**

(71) Anmelder: **BEHRINGWERKE Aktiengesellschaft, Postfach 1140, D-3550 Marburg 1 (DE)**

(72) Erfinder: **Kolar, Cenek, Dr., Deutschhausstrasse 20, D-3550 Marburg 1 (DE)**
Erfinder: **Seiler, Friedrich Robert, Dr., Oberer Eichweg 10, D-3550 Marburg 1 (DE)**
Erfinder: **Knödler, Ursula, Im Winkel 6, D-3557 Ebsdorfergrund 8 (DE)**

(74) Vertreter: **Becker, Heinrich Karl Engelbert, Dr. et al, HOECHST AKTIENGESELLSCHAFT Central Patent Department P.O. Box 80 03 20, D-6230 Frankfurt am Main 80 (DE)**

(54) **Glycopeptide, Verfahren zu ihrer Herstellung und ihre Verwendung.**

(57) Es werden Verbindungen der allgemeinen Formel I, II oder III beschrieben

$R^1–A^1–T–NH–R^2–COR^3$ I

$R^1–A^1–T–A^2–NH–R^2–COR^3$ II

$R^1–A^1–T–A^2–A^3–T–A^4–NH–R^2–COR^3$ III

worin

$R^1$ ein Wasserstoffatom, eine zum Schutz von Aminogruppen übliche Alkyloxycarbonyl- oder Arylalkyloxycarbonylgruppe oder $CH_3–(CH_2)_m–CO$ mit m = 0–16,

$R^2$ –$(CH)_n$– oder –$(CHOH)_n$– CO mit m = 0–16,

$R^3$ eine Hydroxygruppe, O-Alkyl- oder O-Arylalkylschutzgruppe, eine carboxygruppenaktivierende Gruppe, einen Kephalinrest, ein NH-gruppentragender Aminosäurerest von Oligo- oder Polypeptiden oder Proteinen oder ein Träger,

$A^1$, $A^2$, $A^3$ oder $A^4$ einen Bindestrich oder ein in Glycophorin A vorkommender Aminosäurerest wie Ala, Val, Leu, Ile, Ser, Pro, Glu oder Arg, bei dem gegebenenfalls die reaktionsfähigen Gruppen, die nicht in der Peptidbindung beteiligt sind, durch Schutzgruppen geschützt sind, und der Rest T

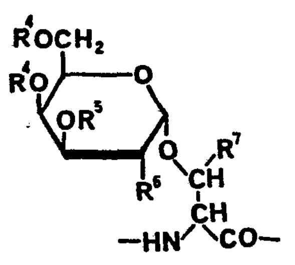

T

in dem

$R^4$ ein Wasserstoffatom oder eine Acylschutzgruppe,

$R^5$ ein Wasserstoffatom, eine Acylschutzgruppe oder der 2,3,4,6-Tetra-O-acetyl-beta-D-galactopyranosyl- oder beta-D-Galactopyranosylrest,

$R^6$ $N_3$, $NH_2$ oder NHAc und

$R^7$ H oder $CH_3$, bedeuten, sind,

sowie ein Verfahren zu ihrer Herstellung und ihre Verwendung, gebunden an einen Träger, als künstliche Antigene, Glycolipide oder Immunadsorbenzien.

EP 0 175 290 A2

BEHRINGWERKE AKTIENGESELLSCHAFT HOE 84/B 020 Dr.HA

# Glycopeptide, Verfahren zu ihrer Herstellung und ihre Verwendung

Die Erfindung betrifft Verbindungen der allgemeinen Formel I, II oder III

$R^1-A^1-T-NH-R^2-COR^3$ I

$R^1-A^1-T-A^2-NH-R^2-COR^3$ II

$R^1-A^1-T-A^2-A^3-T-A^4-NH-R^2-COR^3$ III

worin

$R^1$ ein Wasserstoffatom, eine zum Schutz von Aminogruppen übliche Alkyloxycarbonyl- oder Arylalkyloxycarbonylgruppe oder $CH_3-(CH_2)_m-CO$ mit $m = 0-16$,

$R^2$ $-(CH_2)_n-$ oder $-(CHOH)_n-$ mit $n = 1-10$,

$R^3$ eine Hydroxygruppe, O-Alkyl- oder O-Arylalkylschutzgruppe, eine carboxygruppenaktivierende Gruppe, einen Kephalinrest, ein NH-gruppentragender Aminosäurerest von Oligo- oder Polypeptiden oder Proteinen oder ein Träger,

$A^1$, $A^2$, $A^3$ oder $A^4$ einen Bindestrich oder ein in Glycophorin A vorkommender Aminosäurerest wie Ala, Val, Leu, Ile, Ser,Pro, Glu oder Arg, bei dem gegebenenfalls die reaktionsfähigen Gruppen, die nicht in der Peptidbindung beteiligt sind, durch Schutzgruppen geschützt sind, und der Rest T

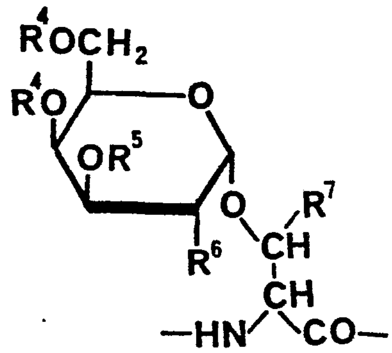

T

bedeuten, in dem

$R^4$ ein Wasserstoffatom oder eine Acylschutzgruppe,

$R^5$ ein Wasserstoffatom, ein Acylschutzgruppe oder der 2,3,4,6-Tetra-0-acetyl-beta-D-galactopyranosyl- oder beta-D-Galactopyranosylrest,

$R^6$ $N_3$, $NH_2$ oder NHAc und

$R^7$ ein Wasserstoffatom oder eine Methylgruppe sind,

sowie ein Verfahren zu ihrer Herstellung und ihre Verwendung, gebunden an einen Träger, als künstliche Antigene, Glycolipide oder Immunadsorbenzien.

Bei Behandlung von Erythrozyten der M-oder N-Blutgruppe mit Neuraminidase kommt es zur Freilegung des sogenannten Thomsen-Friedenreich(T)-Antigens. Dieses T-Antigen wurde als die Disaccharid-Einheit ß-D-Gal-(1-3)-alpha-D-GalNAc, die über L-Serin- oder L-Threonin-Reste in der Polypeptid-Kette der Proteine eingegliedert ist, charakterisiert. Durch die Abspaltung der ß-0-glycosidisch gebundenen Galacto-Einheit erhält man das $T_N$-Antigen.

Die T- oder $T_N$-antigenen Strukturen liegen zum Beispiel in dem Hauptglycoprotein der Erythrozyten-Membran - dem Glycophorin - als "Kryptantigene" vor.

Im Tumorgewebe verschiedener Krebsarten konnte Springer (Naturwissenschaften (1983) 70, 369) jedoch auch T- und $T_N$-antigene Strukturen nachzuweisen, so daß diese als tumor-assoziierte Antigene anzusehen sind.

Der Kohlenhydratanteil der T- und $T_N$-antigenen Strukturen ist zwar charakterisiert, aber nicht der Oligopeptidanteil.

Uhlenbruck et al., Immunbiol. (1983), 165, 147 haben bewiesen, daß für die Spezifität der T-Determinante eine Glycopeptid-Struktur verantwortlich ist. Da hierbei aus Glycophorin isolierte Glycopolypeptide verwendet wurden, konnte die T- und $T_N$-Spezifität nicht geklärt werden. Durch die chemische Synthese von relevanten Glycopeptiden, deren serologische und immunchemische Eigenschaften mit denen der natürlichen T-spezifischen Strukturen vergleichbar sind, wäre es möglich, die vollständige Struktur der Thomsen-Friedenreich-Determinante und der T-spezifischen tumor-assoziierten Determinante zu charakterisieren.

Für die Herstellung von Glycopeptid-Inhibitoren, künstlichen Antigenen oder Immunadsorbenzien mit Strukturen der T- und $T_N$-spezifischen Determinanten werden chemische Verbindungen mit diesen Strukturmerkmalen gebraucht, die mit geeigneten Trägermolekülen umgesetzt werden können. Es wäre möglich mit synthetischen T- und $T_N$-spezifischen Antigenen zu entsprechenden Antikörpern zu gelangen. Diese spezifischen Antikörper wären zur Diagnostik und Früherkennung des Krebses verwendbar.

Bei die Herstellung von Glycopeptiden oder synthetischen Antigenen bestehen meist die Schwierigkeiten in der Schutzgruppenchemie der Kohlenhydrate und Peptide. Ferrari und Pavia (Carbohydrate Research (1980), 79, C1-C7) konnten zwar ein alpha-D-GalNAc-L-Serin oder L-Threonin-Segment synthetisieren, aber die Entfernung einer Methylesterschutzgruppe gelang nicht, womit dieses Segment zur Herstellung von Glycooligopeptiden ungeeignet ist. Paulsen (Chemical Society Reviews (1984), Vol. 13, No.1, 15) und Sinay et al. (Carbohydrate Research (1983), 116/2, C9) konnten zwar terminale Glycopeptide als Segmente des Glycophorins synthetisieren, aber diese Glycopeptide entsprechen nicht der T- und $T_N$-Spezifität, wie Uhlenbruck nachweisen konnte.

Überraschenderweise hat es sich jetzt gezeigt, daß die ungeschützten Glycopeptide der Formel I und II und insbesondere der Formel III serologische T- bzw. $T_N$-Aktivität aufweisen, die dem Asialoglycophorin vergleichbar ist. Es hat sich weiterhin überraschenderweise gezeigt, daß die Glycopeptid-Haptene, die zwei Esterguppen beinhalten, selektiv über die Carboxygruppe des Spacers an Trägermoleküle koppelbar sind. Hiermit eröffnet sich ein neuer Weg zur Herstellung von komplexen Glycopeptpid-Antigenen.

Die vorliegende Erfindung hatte sich zur Aufgabe gestellt, kopplungsfähige Glycopeptide herzustellen, aus denen künstliche T- und $T_N$-aktive Antigene, Immunadsorbenzien oder Glycolipide hergestellt werden können, indem sie kovalent an Träger gebunden werden. Gelöst wird diese Aufgabe durch die Herstellung von Verbindungen der allgemeinen Formeln I, II und III und den oben angegebenen Definitionen und ihre Bindung an Träger.

Bevorzugt sind Verbindungen, die als Teilstrukturen im Glycophorin vorliegen, der

Formel V $R^1$-Leu-T-$NH(CH_2)_5$-$COR^3$
Formel VI $R^1$-Ser-T-$NH(CH_2)_5$-$COR^3$
Formel VII $R^1$-Ile-T-Ser-$NH(CH_2)_5$-$COR^3$
Formel VIII $R^1$-Pro-T-Ala-$NH(CH_2)_5$-$COR^3$
Formel IX $R^1$-Val-T-Glu($R^9$)-$NH(CH_2)_5$-$COR^3$
Formel X $R^1$-Ile-T-Val-$NH(CH_2)_5$-$COR^3$
Formel XI $R^1$-Arg($R^{10}$)-T-Val-$NH(CH_2)_5$-$COR^3$
Formel XII $R^1$-Ala-T-Pro-$NH(CH_2)_5$-$COR^3$
Formel XIII $R^1$-Val-T-Glu($R^9$)-Ile-T-Val-$NH(CH_2)_5$-$COR^3$
Formel XIV $R^1$-Ile-T-Val-Arg($R^{10}$)-T-Val-$NH(CH_2)_5$-$COR^3$

worin
$R^1$ ein Wasserstoffatom oder eine Acetylgruppe,
$R^3$ eine Hydroxygruppe, $OCH_3$, O-tert.Butyl, O-Benzyl, ein Aktivester, ein Polylysin-, Protein- oder Kephalin-Rest, ein aminogruppentragendes Gel oder ein Träger,
$R^9$ ein Wasserstoffatom oder eine Schutzgruzppe für Carboxygruppen, wie die Methyl-, Benzyl oder tert.Butylgruppe,
$R^{10}$ ein Wasserstoffatom oder eine Schutzgruppe für Aminogruppen, wie die $NO_2$-Gruppe und
T die vorher angegebene Bedeutung besitzt,
bedeuten.

Das Verfahren zur Herstellung einer der Verbindungen der Formeln I, II und III ist dadurch gekennzeichnet, daß man a) eine Verbindung der allgemeinen Formel XV

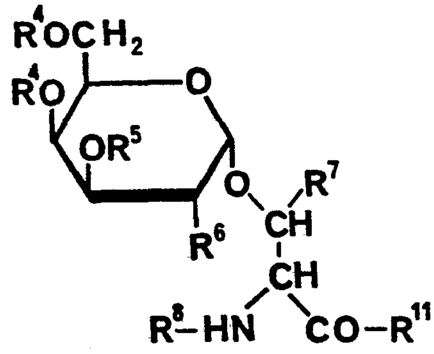

XV

worin

$R^4$ eine Acylschutzgruppe, bevorzugt eine Acetyl- oder Benzoylgruppe,

$R^5$ eine Acylschutzgruppe oder ein 2,3,4,6-Tetra-O-acetyl-beta-D-galactopyranosylrest,

$R^6$ $N_3$ oder NHAc,

$R^7$ H oder $CH_3$,

$R^8$ eine alpha,alpha-Dimethyl-3,5-dimethoxy-benzyloxycarbonyl-, Benzyloxycarbonyl- oder 9-Fluorenylmethyloxycarbonylgruppe und

$R^{11}$ $O-CH_2Ph$ bedeuten,

in Gegenwart eines Hydrierungskatalysators, wie Palladium/Kohle und eines organischen Lösungsmittels wie Methanol, Essigsäureethylester oder Diethylether bei Raumtemperatur hydriert und die gegebenenfalls freie alpha-Aminogruppe des Serins oder Threonins mit einer Alkyloxycarbonyl- oder Arylalkyloxycarbonyl-Schutzgruppe, bevorzugt DDZ, Z, BOC und Fmoc, blockiert, wobei eine Verbindung der allgemeinen Formel XV, worin die Reste $R^4$, $R^5$, $R^6$ und $R^7$ die oben angegebene Bedeutung beibehalten und $R^8$ eine DDZ-, Z-, BOC- oder Fmoc-Gruppe und $R^{11}$ eine Hydroxygruppe ist, umsetzt,

b) das Produkt von Stufe a) nach einem in der Peptidchemie üblichen Kondensationsverfahren wie dem Dicyclohexylcarbodiimid- oder Aktivester-Verfahren oder unter Anwendung von Succinimid- oder p-Nitrophenylestern oder von gemischten Anhydriden mit einer Verbindung der allgemeinen Formel XVI, die eine freie Aminogruppe aufweist,

$$H-A-NH-R^2-COR^3 \qquad XVI$$

worin

A ein Bindestrich oder ein Aminosäurerest, bevorzugt Ala, Val, Pro, Ser, (Benzyl)Ser, Glu(gamma-tert.Butyl) oder Glu(gamma-Bn),

$R^2$ $-(CH_2)_n-$, n = 1-10 und

$R^3$ $OCH_3$, O-Benzyl oder O-tert.-Butyl bedeuten,

oder mit einer Verbindung der allgemeinen Formel XVII

$H-A^2-A^3-O$-Benzyl XVII

worin

$A^2$ Ala, Val, Pro, Ser, (Benzyl)Ser, Glu(gamma-tert.Butyl), Glu(gamma-Methyl) oder Glu(gamma-Bn),

$A^3$ ein Bindestrich oder ein Aminosäurerest Ile oder $(NO_2)$Arg ist,

zu einer Verbindung der allgemeinen Formel XV,

worin

die Reste $R^4$, $R^5$, $R^6$, $R^7$ und $R^8$ unverändert sind, und

$R^{11}$ $A-NH-R^2-COR^3$ oder $A^2-A^3-O$-Benzyl bedeuten, wobei die Reste A, $A^2$, $A^3$, $R^2$ und $R^3$ unverändert sind,

umsetzt,

c) in einem Produkt von Stufe b) in an sich bekannter Weise die Schutzgruppe DDZ, BOC, Z oder Fmoc selektiv hydrolytisch oder hydrogenolytisch abspaltet, und das entstandene alpha-aminogruppentragende Zwischenprodukt mit einer Verbindung der allgemeinen Formel XVIII

$R^1-A^1-OR^{12}$ XVIII

worin

$R^1$ eine Acetylgruppe oder eine Schutzgruppe wie DDZ-, BOC-, Z- oder Fmoc-Gruppe,

$A^1$ ein Rest einer der Aminosäuren Ala, Val, Ile, Pro, Arg oder $(NO_2)$Arg und

$R^{12}$ ein Wasserstoffatom, ein Aktivesterrest wie N-Succinylimid- oder p-Nitrophenyl-Rest bedeuten,

nach einem in der Peptidchemie üblichen Kondensationsverfahren zu einer Verbindung der allgemeinen Formel I oder II,

worin

$R^1$ eine Acetylgruppe oder eine DDZ-, BOC, Z- oder Fmoc-Gruppe,

$R^2$ $-(CH_2)_n-$, n = 1-10,

$R^3$ $OCH_3$, O-Benzyl oder O-tert.-Butyl

$A^1$ einer der angegebenen Aminosäure-Reste und

T ein Rest der Formel XV mit der unter Verfahrensstufe a) bezeichneten Bedeutung,

wobei $R^8$ und $R^{11}$ zusammen ein Bindestrich sind,

oder zu einer Verbindung der allgemeinen Formel XIX

$$R^1\text{-}A^1\text{-}T\text{-}A^2\text{-}A^3\text{-O-Benzyl} \qquad \text{XIX}$$

worin $R^1$, $A^1$, $A^2$ und $A^3$ und T die zuletzt angegebene Bedeutung besitzen, umsetzt,

d) in dem Produkt der allgemeinen Formel XIX die Benzylgruppe selektiv hydrogenolytisch abspaltet und nach einer der in der Peptidchemie üblichen Kondensationsmethoden mit einer Verbindung der allgemeinen Formel XX

$$H\text{-}A^3\text{-}T\text{-}A^4\text{-NH-}R^2\text{-COR}^3 \qquad \text{XX}$$

worin

$A^3$, $A^4$, T, $R^2$ und $R^3$ die zuletzt genannte Bedeutung besitzen zu einer Verbindung der allgemeinen Formel III umsetzt,

e) in einem Produkt von Stufe c) oder d) nach in der Kohlenhydratchemie oder Peptidchemie üblichen Verfahren die Schutzgruppen entfernt und die freie Aminogruppe am

Galactosaminyl-Rest selektiv acetyliert, wobei ein Produkt der allgemeinen Formel I, II und III entsteht, worin

$R^1$ eine Acetylgruppe oder eine Schutzgruppe,

$R^2$ $-(CH_2)_n-$ mit n= 1-10,

$R^3$ eine Hydroxygruppe,

T der eingangs genannte Rest, in dem

$R^4$ ein Wasserstoffatom,

$R^5$ ein Wasserstoffatom oder ein ß-D-Galactopyranosylrest,

$R^6$ NHAc und $R^7$ H oder $CH_3$ sind, bedeuten und

$A^1$, $A^2$, $A^3$ und $A^4$ die zuletzt genannte Bedeutung besitzen,

f) ein Produkt von Stufe e) in an sich bekannter Weise nach einem üblichen Kondensationsverfahren mit einer aminogruppentragenden polymeren Verbindung oder Träger, wie Polylysin, Kephalin, einem Protein oder Gel zu einem synthetischen Glycolipid, Antigen oder Immunadsorbens umsetzt, und

g) gegebenenfalls in einem Produkt von Stufe f) in an sich bekannter Weise nach in der Peptidchemie üblichen Entblockierungsverfahren die restlichen Schutzgruppen entfernt.

Die Glycopeptide und Glycopeptidderivate der allgemeinen Formeln I, II und III werden nach einem Verfahren hergestellt, wie es üblicherweise in der Peptidchemie, zum Beispiel in Houben-Weyl, Methoden der organischen Chemie, Band XV/1 und 2, angewandt wird.

Die Aktivierung der Carboxygruppe kann erreicht werden zum Beispiel durch Umwandlung der Carboxygruppe in ein Säurehalogenid, ein Azid, Anhydrid, Imidazolid oder einen aktivierten Ester, wie den N-Hydroxysuccinimidester oder den p-Nitrophenylester.

Die Aminogruppe kann aktiviert werden durch Umwandlung in ein Phosphitamid oder nach dem Phosphorazo-Verfahren.

Die üblichen Verfahren für die oben angegebenen Kondensationsreaktionen sind: Das Carbodiimidverfahren, das Azidverfahren, das gemischte Anhydridverfahren und das Verfahren der aktivierten Ester, wie sie beschrieben sind in "The Peptides", Band 1, 1965 (Academic Press).

Die reaktionsfähigen Gruppen, die nicht an der Kondensationsreaktion teilnehmen sollen, werden durch Schutzgruppen geschützt, die leicht wieder entfernt werden können, zum Beispiel durch Hydrolyse oder Reduktion. Schutzgruppen sind beispielsweise ebenfalls in "The Peptides" beschrieben.

Es ist besonders vorteilhaft, auch die Hydroxygruppe des Serin-Restes zu schützen. Schutzgruppen in diesem Zusammenhang sind die Benzyl- oder tert.-Butylgruppe. Es ist besonders vorteilhaft, auch die Guanidingruppe von Arginin zu schützen. Eine übliche Schutzgruppe in diesem Zusammenhang ist die Nitrogruppe.

Es ist vorteilhaft, auch die omega-Carboxygruppe von Glutaminsäure zu schützen. Übliche Schutzgruppen in diesem Zusammenhang sind eine tert.-Butyl-carbonyl-, Benzyl- oder Methylgruppe.

Die Schutzgruppen können nach üblichen Verfahren abgespalten werden, je nach Art der jeweiligen Gruppe zum Beispiel mit Trifluoressisäure oder durch milde Reduktion, zum Beispiel mit Wasserstoff und einem Katalysator wie Palladium, oder mit HBr in Eisessig.

Die Schutzgruppen am Kohlenhydratsegment können je nach Art der Schutzgruppe nach üblichen Verfahren abgespalten

werden (Angew. Chem. (1982), 94, 184; Carbohydr. Res. (1983), 116, C9-C12). Die Azidogruppe kann durch milde Reduktion zum Beispiel mit Wasserstoff und einem Katalysator, wie Palladium, oder mit Natriumborhydrid in Gegenwart von Nickel (II)-chlorid, zu einer Aminogruppe umgesetzt werden, die durch Acetylierung z.B. mit Acetanhydrid in Methanol in die Acetylaminogruppe überführt wird. Die O-Acylschutzgruppen werden vorteilhaft im basischen Milieu zum Beispiel mittels Natriummethylat in Methanol oder Natriumhydroxyd oder Natriumcarbonat in Methanol abgespalten.

Die Glycopeptide der Formeln I, II und III werden nach verschiedenen üblichen Verfahren an der Carboxygruppe des "Spacerteils" in Aktivesterderivate überführt wie den N-Hydroxysuccinimidester oder den p-Nitrophenylester und an einen Träger, der eine oder mehrere Aminogruppen besitzt, gekoppelt. Es ist hierbei besonders vorteilhaft, die reaktionsfähigen Gruppen eines Glycopeptides der Formeln I, II und III, die nicht an der Kondensationsreaktion teilnehmen sollen, durch Schutzgruppen zu schützen. Vor allem soll die alpha-Aminogruppe der terminalen Aminosäure des Glycopeptides mit einer Schutzgruppe wie der 9-Fluorenylmethyloxycarbonyl (Fmoc)-, alpha,alpha-Dimethyl-3,5-dimethoxybenzyloxycarbonyl (DDZ)- oder tert.-Butyloxycarbonyl (BOC)-Gruppe geschützt sein. Die Schutzgruppen können wieder leicht entfernt werden zum Beispiel durch Hydrolyse oder Photolyse.

Die Bindung von Haptenen an Träger ist zum Beispiel in DE 32 30 427 beschrieben. Träger sind beispielsweise Proteine, bevorzugt Human- oder Rinderserum-Albumin, Glycoproteine, Polymere wie Polylysin oder Poly(glycyllysin), aktivierte Gele, die Amino-, Glycidyl-, 2-Aminoethylamino- oder Aktivester-Gruppen tragen, Bromcyanaktivierte Polysaccharide oder Polysaccharid-Gele oder Lipide, bevorzugt Kephaline oder aminierte Phospholipide.

Diese trägergebundenen Glycopeptid-Derivate können als künstliche Antigene, Glycolipide oder Immunadsorbentien verwendet werden.

Bezüglich der verschiedenen in der Beschreibung, den Beispielen und den Patentansprüchen verwendeten Abkürzungen ist folgendes zu bemerken:

I. Wenn bei Aminosäureresten keine optische Konfiguration angegeben ist, ist die L-Form gemeint;

II. die folgenden Abkürzungen werden verwendet, um Schutzgruppen oder aktivierende Gruppen zu bezeichnen

| | |
|---|---|
| Adoc | = Adamantyloxycarbonyl |
| Fmoc | = 9-Fluorenylmethyloxycarbonyl |
| DDZ | = alpha,alpha-Dimethyl-3,5-dimethoxy-benzyloxycarbonyl |
| Z | = Benzyloxycarbonyl |
| BOC | = tert.-Butyloxycarbonyl |
| $Bu^tt$ | = tert.-Butyl |
| Me | = Methyl |
| OPN | = p-Nitrophenyl |
| Bn | = Benzyl |
| Bz | = Benzoyl |
| Ac | = Acetyl |
| DDBn | = alpha,alpha-Dimethyl-3,5-dimethoxy-benzyl |
| Gal | = D-Galactopyranosyl |
| GalNAc | = N-Acetyl-D-galactopyranosyl |

Die Struktur der folgenden Verbindungen wurde $^1H$- und $^{13}C$-NMR- sowie IR-spektroskopisch sowie mittels Elementaranalyse bestimmt. Die optischen Drehwerte wurden ebenfalls bestimmt.

Der Verlauf der Reaktionen sowie die resultierenden Produkte wurden dünnschichtchromatographisch und mit der HPLC-Technik untersucht.

Die folgenden Beispiele erläutern die Erfindung näher, ohne sie darauf zu beschränken.

Beispiel 1

Herstellung von Verbindungen der allgemeinen Formel I

$$R^1-A^1-T-NH-(CH_2)_5-COR^3 \quad I$$

In dem folgenden Schema 1 ist die Herstellung der Verbindungen 7 und 8 beschrieben. Die allgemeinen Arbeitsvorschriften sind in dem folgenden experimentellen Teil beschrieben.

N-Acetyl-L-alanyl-(O-(2-acetamido-2-desoxy-alpha-D-galactopyranosyl)-L-threonyl-N-(5-methoxycarbonyl)-n-pentylamid (Verbindung 7)

L-Alanyl-(O-(2-acetamido-2-desoxy-alpha-D-galactopyranosyl)-L-threonyl-N-(5-methoxycarbonyl)-n-pentylamid (Verbindung 8)

Ausgehend von einer Verbindung der allgemeinen Formel XV wurden, wie im Schema 1 gezeigt, folgende Verbindungen der Formel I hergestellt.

0 175 290

| Verbindung Nr. | $R^1$ | $A^1$ | T | $R^3$ |
|---|---|---|---|---|
| 9 | H | Leu | αGalNAc-Ser | OBn |
| 10 | Ac | Leu | αGalNAc-Ser | OBn |
| 11 | H | Leu | βGal(1-3)αGalNAc-Ser | $OCH_3$ |
| 12 | Ac | Leu | βGal(1-3)αGalNAc-Ser | $OCH_3$ |
| 13 | H | Ser | βGal(1-3)αGalNAc-Ser | $OCH_3$ |
| 14 | Ac | Ser | βGal(1-3)αGalNAc-Ser | $OCH_3$ |

Schema 1 (AAV = Allgemeine Arbeitsvorschrift)

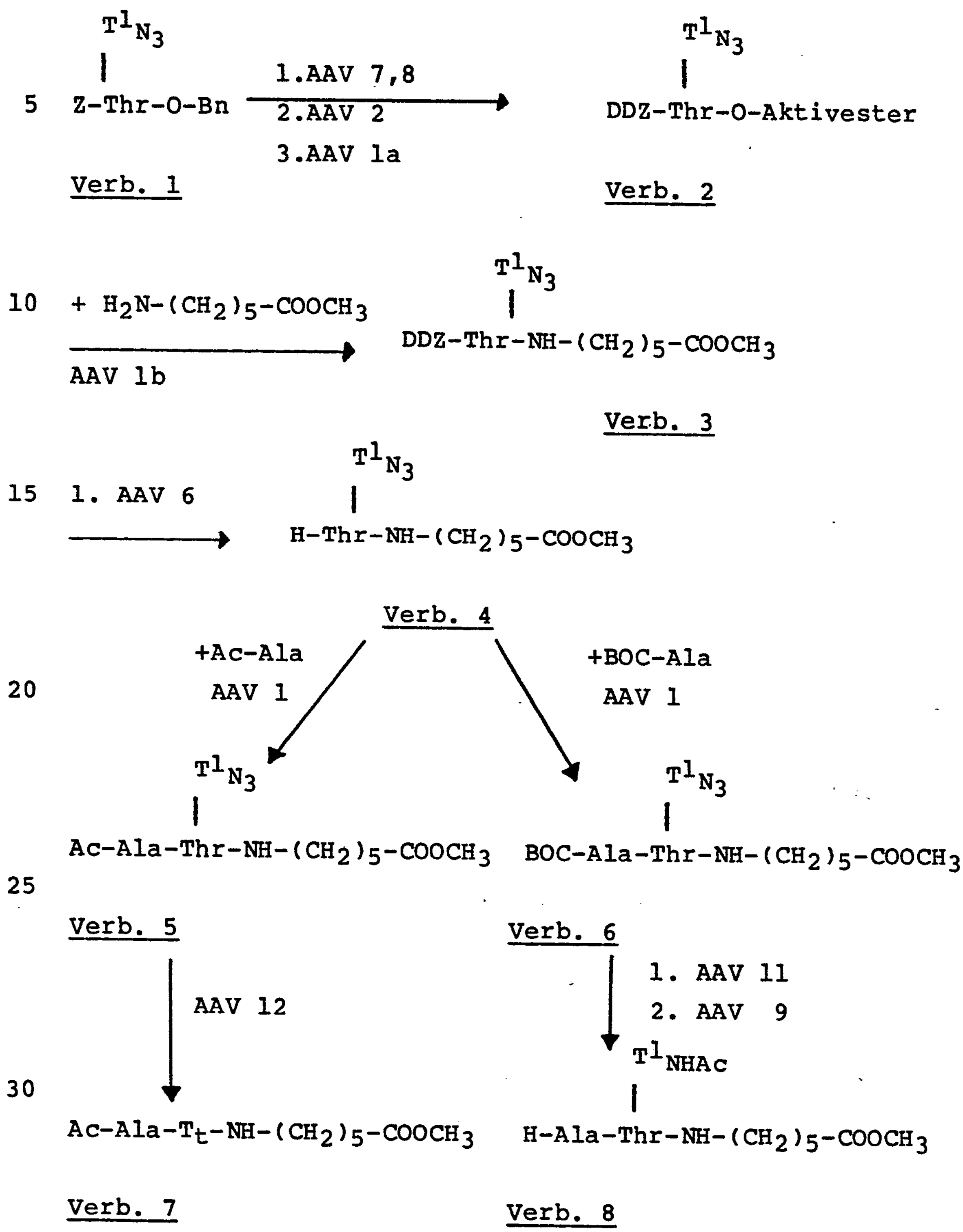


Verb. 1, Verb. 2, Verb. 3, Verb. 4, Verb. 5, Verb. 6, Verb. 7, Verb. 8

$T^1N_3$ = O-(3,4,6-Tri-O-acetyl-2-azido-2-desoxy-alpha-D-galactopyranosyl)

$T^1_{NHAc}$ = O-(2-Acetamido-3,4,6-tri-O-acetyl-2-desoxy-alpha-D-galactopyranosyl)

$T_t$ = O-(2-Acetamido-2-desoxy-alpha-D-galactopyranosyl)-L-threonyl

Beispiel 2

Herstellung von Verbindungen der allgemeinen Formel II

$$R^1\text{-}A^1\text{-}T\text{-}A^2\text{-}NH\text{-}(CH_2)_5\text{-}COR^3 \qquad II$$

In dem folgenden Schema 2 ist die Herstellung der Verbindungen 21 und 22 beschrieben.

L-Isoleucyl-(O-(3-O-(2,3,4,6-tetra-O-acetyl-ß-D-galactopyranosyl)-2-acetamido-4,6-di-O-benzoyl-2-desoxy-alpha-D-galactopyranosyl)-L-seryl-L-valyl-N-(5-benzyloxycarbonyl)-n-pentylamid (Verbindung 21)

N-Acetyl-L-isoleucyl-(O-(2-acetamido-2-desoxy-3-O-(ß-D-galactopyranosyl)-alpha-D-galactopyranosyl)-L-valyl-N-(5-benzyloxycarbonyl)-n-pentylamid (Verbindung 22)

Ausgehend von einer Verbindung der allgemeinen Formel XV wurden, wie in Schema 2 gezeigt, folgende Verbindungen der Formel II hergestellt:

| Verbindung Nr. | $R^1$ | $A^1$ | T | $A^2$ | $R^3$ |
|---|---|---|---|---|---|
| 23 | Ac | Ile | αGalNAc-Ser | Ser | $OCH_3$ |
| 24 | Ac | Ile | ßGal(1-3)αGalNAc-Ser | Ser | $OCH_3$ |
| 25 | Ac | Pro | ßGal(1-3)αGalNAc-Thr | Ala | OBn |
| 26 | H | Val | ßGal(1-3)αGalNAc-Ser | Glu(γ-$But^t$) | OBn |
| 27 | Ac | Val | ßGal(1-3)αGalNAc-Ser | Glu(γ-$But^t$) | OBn |
| 28 | Ac | Val | ßGal(1-3)αGalNAc-Ser | Glu | OBn |
| 29 | Ac | Arg | ßGal(1-3)αGalNAc-Thr | Val | $OCH_3$ |
| 30 | Ac | Ala | ßGal(1-3)αGalNAc-Thr | Pro | OBn |

Schema 2

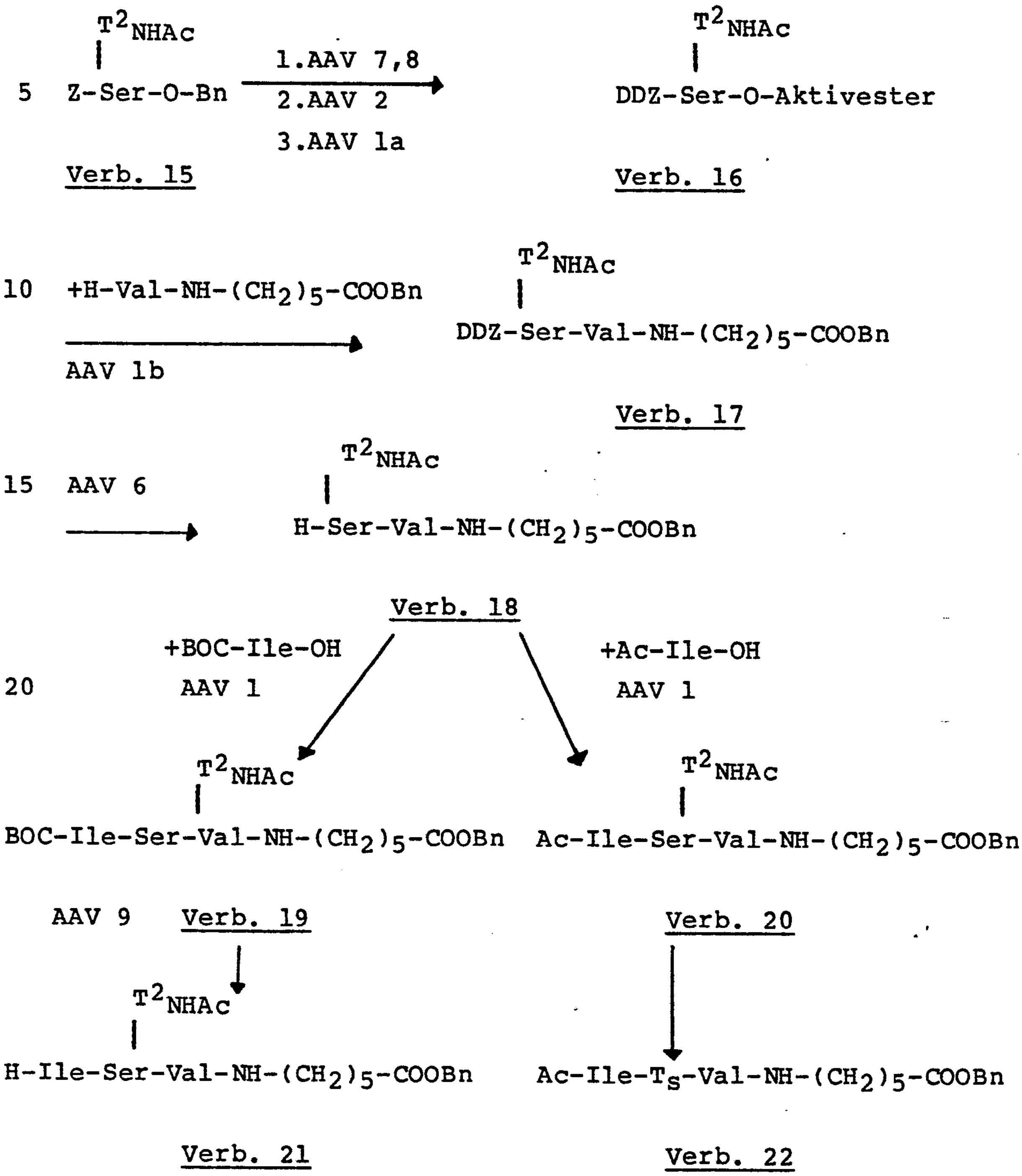


$T^2NHAc$ = O-(3-O-(2,3,4,6-Tetra-O-acetyl-ß-D-galactopyranosyl)-2-acetamido-4,5-di-O-benzoyl-2-desoxy-alpha-D-galactopyranosyl)

$T_s$ = O-(2-acetamido-2-desoxy-3-O-(ß-D-galactopyranosyl)-alpha-D-galactopyranosyl)-L-seryl

Beispiel 3

Herstellung von Verbindungen der allgemeinen Formel III

$$R^1-A^1-T-A^2-A^3-T-NH-(CH_2)_5-COR^3 \qquad III$$

In dem folgenden Schema 3 ist die Herstellung der Verbindung 36 beschrieben.

N-Acetyl-L-valyl-(O-(2-acetamido-2-desoxy-3-O-(ß-D-galactopyranosyl)-alpha-D-galactopyranosyl)-L-seryl-L-glutamyl(gamma-tert.-butyl)-L-isoleucyl-(O-(2-acetamido-2-desoxy-3-O)-(ß-D-galactopyranosyl)-alpha-D-galactopyranosyl))-L-seryl-L-valyl-N-(5-benzyloxycarbonyl)-n-pentylamid (Verbindung 36)

Ausgehend von einer Verbindung der allgemeinen Formel XV wurden, wie in Schema 3 gezeigt, folgende Verbindungen der Formel III hergestellt:

| Verbindung Nr. | $R^1$ | $A^1$ | T | $A^2$ | $A^3$ | $A^4$ | $R^3$ |
|---|---|---|---|---|---|---|---|
| 37 | Ac | Val | Ser<br>ßGal(1-3)αGalNAc | Glu | Ile | Val | OBn |
| 38 | Ac | Val | ßGal(1-3)αGalNAc<br>Ser | Glu($\gamma$-$But^t$) | Ile | Val | OBn |

sowie
N-Acetyl-L-isoleucyl-(O-(2-acetamido-2-desoxy-3-O-(ß-D-galactopyranosyl)-alpha-D-galactopyranosyl)-L-seryl-L-valyl-L-arginyl-(O-(2-acetamido-2-desoxy-3-O)-(ß-D-galactopyranosyl)-alpha-D-galactopyranosyl))-L-threonyl-L-valyl-N-(5-benzyloxycarbonyl)-n-pentylamid (Verbindung 39)

Schema 3

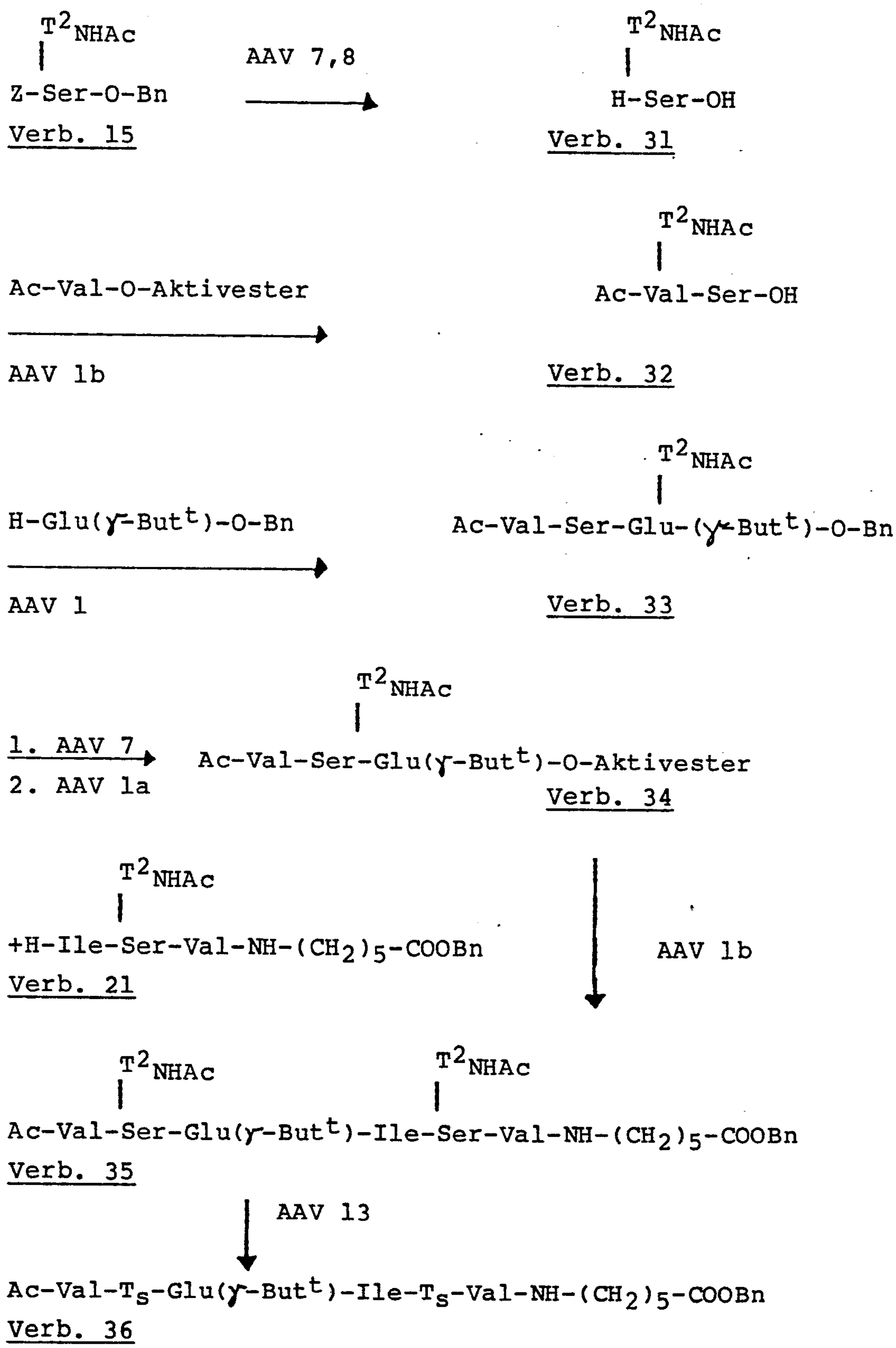


Verb. 15, Verb. 31, Verb. 32, Verb. 33, Verb. 34, Verb. 21, Verb. 35, Verb. 36

$T^2_{NHAc}$ = O-(3-O-(2,3,4,6-Tetra-O-acetyl-ß-D-galactopyranosyl)-2-acetamido-4,5-di-O-benzoyl-2-desoxy-alpha-D-galactopyranosyl)

$T_S$ = O-(2-acetamido-2-desoxy-3-O-(ß-D-galactopyranosyl)-alpha-D-galactopyranosyl)-L-seryl

Beispiel 4

Immobilisierung von Glycopeptid-Verbindungen an aminierten Trägern

Die in den Beispielen 1, 2 und 3 beschriebenen Verbindungen 7, 12, 14, 23 und 24, die als Methylester vorliegen, wurden nach AAV 15 in Carboxylderivate überführt. Die in den Beispielen 1, 2 und 3 beschriebenen Verbindungen 10, 22, 25, 27, 30, 36 und 38, die als Benzylester vorliegen, wurden nach AAV 7 unter Verwendung eines polaren Lösungsmittels, wie Wasser, Methanol oder Methanol/Essigsäureethylester, in Carboxylderivate überführt.

Die resultierenden Glycopeptidderivate, die eine freie Carboxygruppe besitzen, wurden an aminierte Träger nach AAV 16 gekoppelt und von diesen Produkten gegebenenfalls die restliche Schutzgruppe abgespalten. Die üblichen Kopplungsmethoden sind in DE 32 20 426 Al beschrieben.

## Experimenteller Teil

Allgemeine Arbeitsvorschriften (AAV)

AAV 1: Herstellung der Peptidbindung

a) Herstellung eines "Aktivesters"

Das Carbonsäurederivat (3 mmol), das nur eine freie Carboxygruppe aufweist, und bei dem die übrigen reaktiven Gruppen geschützt sind, wurde in 50 ml trockenem Acetonitril gelöst. N-Hydroxysuccinimid (3 mmol) und Dicyclohexyldiimid (3 mmol) wurden unter Rühren zugegeben. Nach 24 h wurde der Reaktionsansatz bei 0°C abfiltriert und im Vakuum eingeengt. Der resultierende Sirup wurde ohne weitere Reinigung im nächsten Reaktionsschritt verwendet.

Häufig verwendete Laufmittelsysteme für Dünnschichtchromatographie: Chloroform/Methanol 9:1, 7:1, 3:1 und 1:1; Chloroform/Essigsäureethylester 1:1.

b) Herstellung einer Peptidbindung

Die Amino-Komponente (3 mmol), die außer einer freien Aminogruppe die übrigen reaktionsfähigen Gruppen geschützt hat, wurde in trockenem Chloroform (25 ml) gelöst und unter Rühren mit etwa 3 mmol 4-(N,N-Dimethyl-amino)-pyridin) auf pH 9 eingestellt. Nach 10 min wurde der in 25 ml trockenem Chloroform gelöste "Aktivester" aus der Stufe a) zugegeben. Nach 24 h wurde der Reaktionsansatz einmal mit 5%-iger Citronensäure ausgewaschen, mit Natriumsulfat getrocknet und im Vakuum eingeengt. Der resultierende Sirup wurde säulenchromatographisch an Kieselgel gereinigt.

Häufig verwendete Laufmittelsysteme für die Chromatographie: Chloroform/Essigsäureethylester 1:1; Chloroform/Aceton 7:1, 4:1 uns 1:1; Chloroform/Methanol 9:1 und 5:1.

<u>AAV 2</u>: Blockierung und Schutz der alpha-Amino-Funktion der Aminosäure-Derivate mit der DDZ-Schutzgruppe

Die DDZ-Schutzgruppe wurde nach einer Vorschrift, die von Ch. Birr in Int.J.Peptide Protein Res. (1979) 13, 287-295 beschrieben ist, in die Aminosäure-Derivate eingeführt.

<u>AAV 3</u>: Blockierung und Schutz der alpha-Amino-Funktion der Aminosäure-Derivate mit der BOC-Schutzgruppe

Die BOC-Schutzgruppe wurde nach den Vorschriften, die in Houben-Weyl, Methoden der organischen Chemie, Band XV/I und II, Hrsg. E. Müller und E. Wünsch 1974, beschrieben sind, in die Aminosäure-Derivate eingeführt.

<u>AAV 4</u>: Blockierung und Schutz der Carboxy- oder alkoholischen Hydroxy-Funktion der Aminosäure-Derivate mit der tert.-Butyl-Schutzgruppe

Die tert.-Butyl-Schutzgruppe wurde nach den Vorschriften, die in Houben-Weyl, Methoden der organischen Chemie, Band XV/I und II, Hrsg. E.Müller und E. Wünsch 1974, beschrieben sind, in die Aminosäure-Derivate eingeführt.

<u>AAV 5</u>: Blockierung und Schutz der Carboxy- oder alkoholischen Hydroxy-Funktion der Aminosäure-Derivate mit der DDBn-Schutzgruppe

Die DDBn-Schutzgruppe wurde in gleicher Weise wie in AAV 4 beschrieben an die Carboxy- oder alkoholische Hydroxy-Funktion der Aminosäure-Derivate eingeführt gebunden.

AAV 6: Selektive hydrolytische Abspaltung der alpha, alpha-Dimethyl-3,5-dimethoxy-benzyloxycarbonyl (DDZ)-Schutzgruppe vom Glycopeptid

Das DDZ-Schutzgruppen-tragende Glycopeptid (3 mmol) wurde in 50 ml 5 Vol.-%iger Trifluoressigsäure in Dichlormethan bei Raumtemperatur gelöst. Nach 30 min Rühren wurde mit N-Methylmorpholin bis pH 7 neutralisiert. Die Mischung wurde einmal mit Eiswasser, dann mit verdünnter Salzsäure gewaschen. Die organische Phase wurde mit Natriumsulfat getrocknet und in vacuo zum Sirup eingeengt. Das Produkt lag als Hydrochlorid vor.

Eine dünnschichtchromatographische Kontrolle wurde mit folgenden Laufmittelsystemen durchgeführt: Chloroform/Essigsäureethylester 1:1; Chloroform/Aceton 4:1 und 1:1; Chloroform/Methanol 4:1 und 1:1.

AAV 7: Selektive hydrogenolytische Abspaltung der Benzyl-Schutzgruppe am Glycopeptid

Das Benzyl-Schutzgruppen-tragende Glycopeptid (3 mmol) wurde in 70 ml trockenem Essigsäureethylester gelöst und in Gegenwart von 3,5 g 10%-igem Palladium auf Kohle 2 h hydriert. Dann wurde filtriert, nachgewaschen und in vacuo zum Sirup eingeengt. Das resultierende Produkt wurde säulenchromatographisch gereinigt.

Laufmittel für Chromatographie: Chloroform/Aceton 4:1 und 1:1; Chloroform/Methanol 5:1 und 1:1.

AAV 8: Selektive hydrogenolytische Abspaltung der Benzyloxycarbonyl(Z)-Schutzgruppe am Aminosäure-Derivat

Das Z-Schutzgruppen-tragende Produkt (10 mmol) wurde in 50 ml einer Mischung aus Essigsäureethylester und Methanol (1:1) in Gegenwart von 3 g 10%-igem Palladium auf Kohle 1 h hydriert. Dann wurde filtriert, nachgewaschen und in vacuo eingeengt. Das resultierende Sirup wurde in Chloroform aufgenommen und mit verdünnter Salzsäure gewaschen. Die organische Phase wurde mit Natriumsulfat getrocknet und in vacuo zum Sirup eingeengt. Das Produkt, das als Hydrochlorid vorlag, wurde säulenchromatographisch an Kieselgel gereinigt.

Häufig verwendete Laufmittelsysteme für Chromatographie: Chloroform/Aceton 4:1; Chloroform/Methanol 5:1.

AAV 9: Selektive hydrolytische Abspaltung der tert.-Butyloxycarbonyl(BOC)-Schutzgruppe

Das BOC-Schutzgruppen-tragende Produkt (2,6 mmol) wurde in 10 ml 1,2 normalem Chlorwasserstoff in trockenem Eisessig bei Raumtemperatur gelöst. Nach 20 min Rühren wurde der Reaktionsansatz mit 20 ml Ether versetzt und in vacuo eingeengt. Der resultierende Sirup wurde mehrmals mit Toluol versetzt und eingeengt, bis der Geruch von Essigsäure nicht mehr erkennbar war. Das resultierende einheitliche Produkt wurde säulenchromatographisch an Kieselgel gereinigt.

Häufig verwendete Laufmittelsysteme für Chromatographie: Chloroform/Aceton 9:1 und 4:1; Chloroform/Methanol 9:1 und 5:1.

AAV 10: Selektive hydrolytische Abspaltung der alpha-alpha-Dimethyl-3,5-dimethoxy-benzyl(DDBn)-gruppe mit Trifluoressigsäure

a) Das DDBn-schutzgruppen-tragende Glycopeptid (3 mmol) wurde in 50 ml 5 Vol.-%iger Trifluoressigsäure in Dichlormethan bei Raumtemperatur gelöst. Nach 30 min

Rühren wurde mit N-Methylmorpholin neutralisiert. Die Mischung wurde einmal mit Eiswasser, dann mit verdünnter Salzsäure gewaschen. Die organische Phase wurde mit Natriumsulfat getrocknet und in vacuo zum Sirup eingeengt.

b) Die DDBn-Schutzgruppe kann ebenfalls mit Säuren, wie HCl, in wäßrigem Milieu abgespalten werden.

Laufmittelsysteme: Chloroform/Aceton 9:1, 4:1 und 1:1; Chloroform/Methanol 4:1 und 1:1.

<u>AAV 11</u>: Selektive Reduktion der 2-Azido- zur 2-Aminogruppe am Galactose-Baustein mit $NiCl_2$-$NaBH_4$ und anschließende Acetylierung der Aminogruppe zur Acetamidogruppe

Die Azid-Verbindung (1 mmol) wurde in Ethanol (5 ml) und $NiCl_2$-Lösung (5 ml; 4% (w:v) $NiCl_2$ x 6 $H_2O$ in Ethanol und 1% (w:v) Borsäurezusatz) gelöst und mit $NaBH_4$ (1 bis 2 Äquivalente) versetzt. Nach Reaktionsende wurde Pyridin (5 ml) und Acetanhydrid (5 ml) hinzugegeben und bei 20°C 3 bis 24 h gerührt. Danach wurde eingeengt und mit Chloroform/Wasser ausgeschüttelt. Die organische Phase wurde getrocknet und in vacuo eingeengt.

<u>AAV 12</u>: Selektive Hydrogenolyse der 2-Azidogruppe zur 2-Aminogruppe am Galactose-Baustein und anschließende Acetylierung der Aminogruppe zur Acetamidogruppe

a) Reduktion der Azidogruppe zur Aminogruppe
In einer Suspension aus 500 mg 10%-igem Palladium auf Kohle und 100 ml trockenem Methanol wurde ca. 30 min Wasserstoff eingeleitet. Mit einem Gemisch aus Methanol und wäßriger konzentrierter Soda-Lösung (50:1)

wurde der pH-Wert der Suspension auf pH 7 eingestellt. Die Azid-Verbindung (5 mmol), gelöst in wenig Methanol, wurde zugegeben und anschließend 3 h unter Lichtschutz hydriert. Im Verlauf der Hydrierung wurde der pH-Wert kontrolliert und, falls erforderlich, auf pH 7 eingestellt. Dann wurde filtriert, sorgfältig mit Methanol/Ether nachgewaschen und in vacuo zum Sirup eingeeengt.

b) Acetylierung

Das Produkt von Stufe a) wurde in trockenem Methanol gelöst und mit Acetanhydrid (50 mmol) versetzt. Nach 24 h Rühren wurde der Reaktionsansatz in vacuo eingeengt und der resultierende Sirup mehrmals mit Toluol versetzt und in vacuo eingeengt. Das einheitliche Produkt wurde säulenchromatographisch an Kieselgel gereinigt.

Häufig verwendete Laufmittelsysteme zur Chromatographie: Chloroform/Aceton 9:1, 5:1 und 2:1, Chloroform/Methanol 9:1 und 6:1.

Bei der Acetylierung wurde nicht nur die Aminogruppe des Galactose-Bausteines sondern auch, falls vorhanden, die alpha-Aminogruppe der terminalen Aminosäure am Glycopeptid in die Acetamidogruppe überführt.

AAV 13: Abspaltung der O-Acyl-Schutzgruppen am Galactose-Baustein des Glycopeptids

Das Glycopeptidd (2 mmol) wurde in 30 ml Methanol gelöst. Zu der Lösung wurde tropfenweise eine wäßrige konzentrierte Natriumcarbonatlösung zugegeben, bis der pH-Wert 11 betrug. Nach 20 h Rühren wurde der Reaktionsansatz mit aktiviertem Ionenaustauscher neutralisiert und abfiltriert. Die Lösung wurde in vacuo zum Sirup eingeengt

und das verbleibende einheitliche Produkt säulenchromatographisch gereinigt.

Die Abspaltung der Acyl-Schutzgruppen kann statt mit Natriumcarbonat auch mit katalytischen Mengen Natriummethylat durchgeführt werden.

Häufig verwendete Laufmittelsysteme zur Chromatographie: Chloroform/Methanol/Wasser 20:5:0,4 und 4:4:1.

<u>AAV 14</u>: Abspaltung der Ester-gebundenen gamma-tert.-Butylschutzgruppen an Glutaminsäure-haltigen Glycopeptiden

a) Das Glycopeptid (0,7 mmol), das Glutaminsäure-gamma-tert.-Butylester-Bausteine enthält, wurde in 4 ml 90 Vol.-%iger wässriger Trifluoressigsäure gelöst. Nach 50 min bei 20°C wurden 200 ml Diethylether zugegeben, der Niederschlag abzentrifugiert, zweimal mit je 40 ml Diethylether gewaschen und getrocknet.

b) Die Abspaltung der gamma-tert.-Butylschutzgruppe von Glutaminsäure-gamma-tert.-butylester enthaltenden Glycopeptid-Segmenten an wurde, wie in AAV 6 beschrieben, durchgeführt.

Häufig verwendete Laufmittelsysteme zur Chromatographie: Chloroform/Methanol 5:1 und 3:1; Chloroform/Methanol/Wasser 4:4:1.

<u>AAV 15</u>: Spaltung des Methylesters am Spacer des Glycopeptids

Zu einer Lösung der Methylester-Verbindung (0,5 mmol) in 30 ml 1,4-Dioxan/Wasser (9:1) wurden unter Rühren mit

einer 1 normalen Natriumhydroxid-Lösung in Portionen zu 1 ml unter Kontrolle des Laugenverbrauchs (etwa 0,5 mmol NaOH) mit Thymolphthalein als Indikator bei 20°C verseift. Es wurde mit dem Ionenaustauscher Dowex 50 WX-8H$^-$ neutralisiert, filtriert, nachgewaschen und in vacuo eingedampft. Das verbleibende einheitliche Produkt wurde auf einer Säule mit Sephadex G-25 und als Eluationsmittel Methanol/Wasser (1:1) gereinigt und lyophilisiert.

Laufmittelsysteme für Dünnschichtchromatographie: Chloroform/Methanol/Wasser 5:3:0,5 und 4:4:1.

AAV 16: Bindung von eine Spacergruppe tragenden Glycopeptid-Haptenen an aminiierte Träger

Die eine spacergruppe tragenden Glycopeptidverbindungen, die eine freie kopplungsfähige Carboxygruppe aufweisen, wurden entweder direkt mit Carbodiimiden wie zum Beispiel 1-Ethyl-3-(3′-dimethylaminopropyl)-carbodiimid-Hydrochlorid oder als aktivierte Ester wie zum Beispiel N-Hydroxysuccinimid-Derivate an Proteine wie zum Beispiel Rinderserumalbumin, Polypeptide wie Polylysin oder aminiierte Absorbenzien als Trägermaterialien nach bekannten Verfahren gekoppelt.

Bei den Produkten, die gegebenenfalls noch einen geschützten Aminosäure-Baustein, wie L-Glu(gamma-tert.-Butyl) oder L-Glu(gamma-DDBn), tragen, wurde die Schutzgruppe hydrolytisch in Gegenwart von Essigsäure, Trifluoressigsäure oder deren wäßrigen Mischungen abgespalten.

Analysendaten

Verbindung 3

1H-NMR = (400 MHz, $CD_3OD$)
4,78 (d, M-1 J (1,2) = 3,4 Hz)
3,65 (s, $CH_3O$)
1,37 (d, $CH_3$-Thr, J($CH_3$, CH) = 6,7 Hz

Verbindung 4

$(\alpha)_D^{20}$ = + 97,7° (c = 1 in $H_2O$)

Verbindung 7

$(\alpha)_D^{20}$ = + 83° (c = 1 in $H_2O$)

1H-NMR (400 MHz, $D_2O$)
$\delta$ = 4,85 (H-1, J(1,2) = 3,6 Hz)
3,60 ($COOCH_3$)
1,9 ($CH_3O$)
1,32 ($CH_3$-Thr, J($CH_3$, CH) = 6,6 Hz)

Verbindung 11

$(\alpha)_D^{20}$ = + 79,2° (c = 1,2 in $H_2O$)

1H-NMR (400 MHz, $D_2O$)
$\delta$ = 4,77 (d,H-1, J(1,2) = 3,2 Hz)
4,30 (d,H-1', J(1',2') = 7,7 Hz)
3,56 (s,$COOCH_3$)
1,49-1,35 ($CH_2$-Spacer)

Verbindung 13

$(\alpha)_D^{20}$ = + 83,2° (c = 1 in $H_2O$)

1H-NMR (400 MHz, $D_2O$)
$\delta$ = 4,72 (d,H-1, J(1,2) = 3,4 Hz)
4,27 (d,H-1', J(1',2') = 7,8 Hz)

Verbindung 18

$(\alpha)_D^{20}$ = + 63,7° (c = 1 in Chloroform)

1H-NMR (400 MHz, $CDCl_3$):
$\delta$ = 8,12-7,35 (m, Ph)
5,82 (d,NHAc, J(NH,2) = 9,0 Hz)
4,66 (d,H-1', J(1',2') = 7,9 Hz)
3,65 ($CH_3O$)
2,06-1,90 (5xAc)
1,62 und 1,32 ($CH_2$-Spacer)

Verbindung 19

$(\alpha)_D^{20}$ = + 74,0° (c = 1 in $CHCl_3$)

1H-NMR (270 MHz, $CDCl_3$):
$\delta$ = 3,66 (s, $OCH_3$-Spacer)
4,25 (d, H-1' J(1',2') = 7,8 Hz)
4,82 (d, H-1, J(1,') = 3,6 Hz)
5,00 (dd, H-3')
5,10 (dd, H-2')
5,33 (dd, H-4')
5,92 (dd, H-4)

Verbindung 22

$(\alpha)_D^{20}$ = + 72,3° (c = 1 in MeOH/$CHCl_3$)

Verbindung 23

$(\alpha)_D^{20}$ = + 98,3° (c = 1 in $CH_3OH/CHCl_3$ = 3:1)

Verbindung 24

$(\alpha)_D^{20}$ = + 71,7° (c = 1 in $CHCl_3$/MeOH = 2:1)

13C-NMR (90 MHz, $CDCl_3/CD_3OD$ 2:1)

$\delta$ = 174-170 (6xCO)
101,1 (C-1'; ß)
99,0 (C-1; $\alpha$)
39,39 und 39,29 ($CH_2$-Spacer)
36,89 (Ile)
33,91; 28,82 und 26,38 ($CH_2$-Spacer)
22,15 ($CH_3$ Acet)
15,27 ($CH_3$ Ile)
10,89 ($CH_3$ Ile)

Patentansprüche:

1. Verbindung der allgemeinen Formel I, II oder III

$R^1-A^1-T-NH-R^2-COR^3$ I

$R^1-A^1-T-A^2-NH-R^2-COR^3$ II

$R^1-A^1-T-A^2-A^3-T-A^4-NH-R^2-COR^3$ III

worin

$R^1$ ein Wasserstoffatom, eine zum Schutz von Aminogruppen übliche Alkyloxycarbonyl- oder Arylalkyloxycarbonylgruppe oder $CH_3-(CH_2)_m-CO$ mit m = 0-16,

$R^2$ $-(CH_2)_n-$ oder $-(CHOH)_n-$ mit n = 1-10,

$R^3$ eine Hydroxygruppe, O-Alkyl- oder O-Arylalkylschutzgruppe, eine carboxygruppenaktivierende Gruppe, einen Kephalinrest, ein NH-gruppentragender Aminosäurerest von Oligo- oder Polypeptiden oder Proteinen oder ein Träger,

$A^1$, $A^2$, $A^3$ oder $A^4$ einen Bindestrich oder ein in Glycophorin A vorkommender Aminosäurerest wie Ala, Val, Leu, Ile, Ser,Pro, Glu oder Arg, bei dem gegebenenfalls die reaktionsfähigen Gruppen, die nicht in der Peptidbindung beteiligt sind, durch Schutzgruppen geschützt sind, und der Rest T

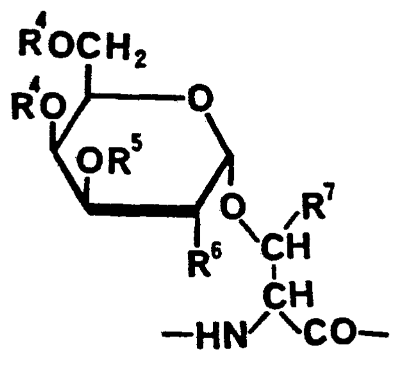

T

 

in dem

$R^4$ ein Wasserstoffatom oder eine Acylschutzgruppe,

$R^5$ ein Wasserstoffatom, ein Acylschutzgruppe oder der 2,3,4,6-Tetra-O-acetyl-beta-D-galactopyranosyl- oder beta-D-Galactopyranosylrest,

$R^6$ $N_3$, $NH_2$ oder NHAc und

$R^7$ ein Wasserstoffatom oder eine Methylgruppe sind,

bedeuten.

2. Verfahren zur Herstellung einer Verbindung nach Anspruch 1, dadurch gekennzeichnet, daß man in einer Verbindung der allgemeinen Formel XV

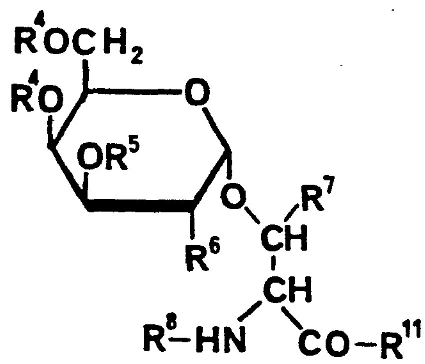

XV

in der

$R^4$ eine Acylschutzgruppe,

$R^5$ eine Acylschutzgruppe oder 2,3,4,6-Tetra-O-acetyl-beta-D-galactopyranosyl,

$R^6$ $N_3$ oder NHAc,

$R^7$ H oder $CH_3$,

$R^8$ eine alpha,alpha-Dimethyl-3,5-dimethoxy-benzyloxycarbonyl, Benzyloxycarbonyl- oder 9-Fluorenylmethyloxycarbonylgruppe und

$R^{11}$ $O-CH_2Ph$ bedeuten,

in Gegenwart eines Hydrierungskatalysators, wie Palladium/Kohle und eines organischen Lösungsmittels wie Methanol, Essigsäureethylester oder Diethylether

bei Raumtemperatur hydriert und die gegebenenfalls freie alpha-Aminogruppe des Serins oder Threonins mit einer Alkyloxycarbonyl- oder Arylalkyloxycarbonyl-Schutzgruppe blockiert, und das Produkt, das nun eine freie Carboxygruppe aufweist, nach einem in der Peptidchemie üblichen Kondensationsverfahren wie dem Dicyclohexylcarbodiimid- oder Aktivester-Verfahren oder unter Anwendung von Succinimid- oder p-Nitrophenylestern oder von gemischten Anhydriden mit einer Verbindung der allgemeinen Formel XVI, die eine freie Aminogruppe aufweist,

$$H\text{-}A\text{-}NH\text{-}R^2\text{-}COR^3 \qquad XVI$$

worin
A ein Bindestrich oder ein Aminosäurerest,
$R^2$ $-(CH_2)_n-$, n = 1-10 und
$R^3$ $OCH_3$, O-Benzyl oder O-tert.-Butyl bedeuten,
oder mit einer Verbindung der allgemeinen Formel XVII

$$H\text{-}A^2\text{-}A^3\text{-}O\text{-}Benzyl \qquad XVII$$

worin
$A^2$ Ala, Val, Pro, Ser, (Benzyl)Ser, Glu(gamma-tert. Butyl), Glu(gamma-Methyl) oder Glu(gamma-Bn),
$A^3$ ein Bindestrich oder ein Aminosäurerest Ile oder $(NO_2)$Arg ist,

zu einer Verbindung der allgemeinen Formel XV,
worin
die Reste $R^4$, $R^5$, $R^6$, $R^7$ und $R^8$ unverändert sind, und
$R^{11}$ $A\text{-}NH\text{-}R^2\text{-}COR^3$ oder $A^2\text{-}A^3$-O-Benzyl bedeuten, wobei die Reste A, $A^2$, $A^3$, $R^2$ und $R^3$ unverändert sind,
umsetzt, und das Produkt, das nach Abspaltung der Schutzgruppe an der alpha-Aminogruppe des Serin- oder Threoninbaustein entsteht, mit einer Verbindung der allgemeinen Formel XVIII

$R^1-A^1-OR^{12}$ XVIII

worin

$R^1$ eine Acetylgruppe oder eine Schutzgruppe wie DDZ-, BOC-, Z- oder Fmoc-Gruppe,

$R^{12}$ ein Wasserstoffatom oder ein Aktivesterrest wie N-Succinimid- oder p-Nitrophenylrest und

$A^1$ ein Rest einer der Aminosäuren Ala, Val, Ile, Pro, Arg oder $(NO_2)$Arg bedeuten,

nach einem in der Peptidchemie üblichen Kondensationsverfahren zu einer Verbindung der allgemeinen Formel I oder II,

worin

$R^1$ eine Acetylgruppe oder eine DDZ-, BOC, Z- oder Fmoc-Gruppe,

$R^2$ $-(CH_2)_n-$, n = 1-10,

$R^3$ $OCH_3$, O-Benzyl oder O-tert.-Butyl

$A^1$ einer der angegebenen Aminosäure-Reste und

T ein Rest der Formel XV,

worin

$R^4$ eine Acylschutzgruppe, bevorzugt eine Acetyl- oder Benzoylgruppe,

$R^5$ eine Acylschutzgruppe oder ein 2,3,4,6-Tetra-O-acetyl-beta-D-galactopyranosylrest,

$R^6$ $N_3$ oder NHAc und

$R^7$ H oder $CH_3$ bedeuten und

$R^8$ und $R^{11}$ zusammen ein Bindestrich sind,

oder mit einer Glycopeptid-Verbindung, die nur eine freie Carboxygruppe aufweist und die nach der Hydrogenolyse einer Benzylesterverbindung der Formel XIX

$R^1-A^1-T-A^2-A^3$-O-Benzyl XIX

$R^1$ eine Acetylgruppe oder eine Schutzgruppe und

$A^1$, $A^2$ und $A^3$ in der L-Konfiguration vorliegende Aminosäurereste, die in Glycophorin enthalten sind und

bei den vorhandene Seitenkettenfunktionen gegebenenfalls durch in der Peptidchemie übliche Schutzgruppen geschützt sind,
T ein Rest der Formel XV
in dem
$R^4$, $R^5$, $R^6$ und $R^7$ die zuletzt genannte Bedeutung besitzen und
$R^8$ und $R^{11}$ Bindestriche darstellen,
nach den in der Peptidchemie üblichen Kondensationsmethoden zu Verbindungen der allgemeinen Formeln I, II und III umgesetzt wird,
worin die Reste $R^1$, $R^2$, $R^3$, $A^1$, $A^2$, $A^3$, $A^4$, und T die zuletzt genannte Bedeutung besitzen, umsetzt, und diese Verbindungen gegebenenfalls in weitere Verbindungen der Formel I, II oder III umwandelt.

3. Verwendung einer Verbindung nach Anspruch 1 gebunden an einen Träger als Antigen, Glycolipid oder Immunadsorbens.